(19) Europäisches Patentamt / European Patent Office / Office européen des brevets

(11) **EP 2 805 943 A1**

(12) **EUROPEAN PATENT APPLICATION**

(43) Date of publication:
**26.11.2014 Bulletin 2014/48**

(51) Int Cl.:
***C07D 311/62*** *(2006.01)* ***C08L 75/04*** *(2006.01)*

(21) Application number: **13168572.9**

(22) Date of filing: **21.05.2013**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Albert-Ludwigs-Universität Freiburg**
**79085 Freiburg (DE)**

(72) Inventors:
- **Garcia-Marrero, Danny-Eugenio**
  **79108 Freiburg im Breisgau (DE)**
- **Glasser, Wolfgang**
  **Richmond, VA 23235 (US)**
- **Laborie, Marie Pierre**
  **79104 Freiburg im Breisgau (DE)**
- **Pizzi, Antonio**
  **88000 Chantraine (FR)**

(74) Representative: **Dr. Langfinger & Partner**
**In der Halde 24**
**67480 Edenkoben (DE)**

(54) **Compositions comprising hydroxypropylated condensed tannin derivatives**

(57) Hydroxypropylated condensed tannin derivatives having a degree of substitution of phenolic hydroxyl groups by hydroxypropyl ether groups in the monomeric unit in the range of from 0.1 to 4.7 and a maximum variation of the degree of substitution of 0.6 or less and a process for their manufacture.

Fig. 1

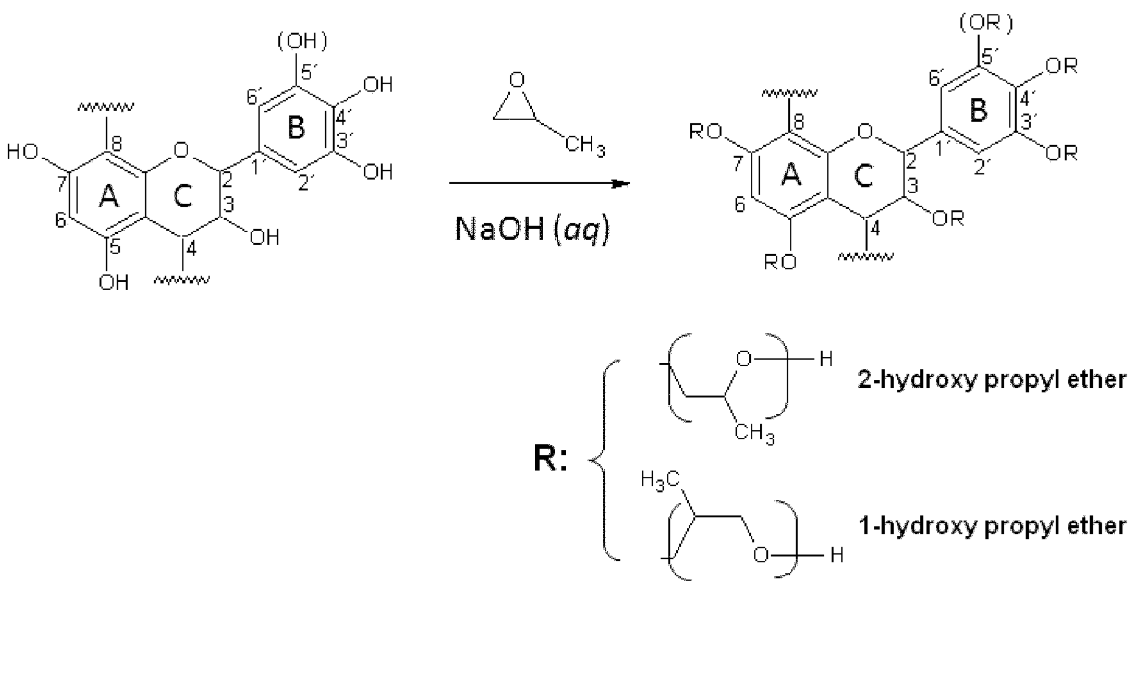


EP 2 805 943 A1

## Description

The present invention relates to compositions comprising modified flavonoid tannin derivatives

Condensed tannins (CTs) are one of the most widely distributed secondary metabolites in the plant kingdom. After lignin, CTs are the most abundant source of natural aromatic macromolecules. The CTs are polyphenols, present in all vascular plants of tropical and subtropical regions, mainly in legumes and conifers exhibiting many eco-physiological functions.

Some tannins are commercially available. The most relevant are from Mimosa (*Acacia mearnsii* formerly *mollissima,* De Wildt), Quebracho (*Schinopsis balansae* and *Schinopsi lorentzii*) and Pine (*Pinus radiata* and *Pinus pinaster*) sources.

Mimosa and Quebracho tannins have encountered much success in biopolymer formulations. However, due to the high reactivity and viscosity of pine tannins, their use in the formulation of polycondensation polymers has been much more limited.

Polyphenol modification by grafting has been carried out repeatedly with lignins as well with other sources.

In analogy to lignin, which constitutes a bio-polyphenolic macromolecule similar to tannin, the chemical derivatization of condensed tannins is expected to offer similar opportunities for property control. Commonly, two routes have been used to introduce ether functionalities into polyphenols: (*i*) the Williamson ether synthesis and (*ii*) the ring opening reaction of epoxides. Simple polyphenols, basically flavonoids with a repeating unit C-15 structure, have been ester- and ether-derivatized with substantial influence of their chemical and physical properties.

The reactions used for the chemical derivatization until today, however, yielded products with a significant variation in the product properties due to the lack of reproducibility of the reactions occurring and the diversity of the possible polyphenolic derivatization methods. It is not yet known which parameter could be used to engineer the product properties. It would be desirable to have condensed tannin derivatives which could be used in industrial processes.

Brief description of the drawings.

Fig. 1 shows the reaction scheme for the reaction of a C-15 flavonoid repeat unit with propylene oxide, where the scheme shows a full derivatization.

Fig. 2 shows the synthesis and isolation scheme of hydroxypropylated tannin derivatives, also termed "hydroxypropyl tannins" (HPT).

Fig. 3 shows 1 H-NMR spectra of the fractions obtained in accordance with Fig. 2.

Fig. 4 shows the yield of reaction products collected in the form of different fractions and estimated yields in relation to the molar ratio of propylene oxide to monomeric tannin unit.

It was an object of the present invention to provide condensed tannin derivatives with good reproducibility of product parameters.

This object has been achieved with the compositions in accordance with claim 1.

Preferred embodiments of the present invention are set forth in the dependent claims and the detailed description hereinafter.

This object is achieved by hydroxypropylated condensed tannin derivatives having obtainable by reacting a bark extract comprising condensed tannin derivatives in an aqueous solvent at a pH in the range of from 9 to 14 and a temperature in the range of from 10 to 50°C with propylene oxide.

The term tannin commonly denominates a polyphenolic compound that binds to and precipitates proteins and various other organic compounds such as e.g. amino acids.

Tannin compounds are widely distributed in many species of plants, where they play the role in protection from predation and perhaps also as pesticides, and implant growth regulation. The destruction or modification of tannins with time plays an important role in the ripening of food and the aging of wine.

The term tannin by extension is also widely applied to large polyphenolic compounds containing sufficient hydroxyls and other suitable groups to form strong complexes with proteins and other macromolecules.

There are two major classes of tannins, so called hydrolyzable tannins and condensed tannins. The flavone basis for the common condensed tannins may be represented by the following formula

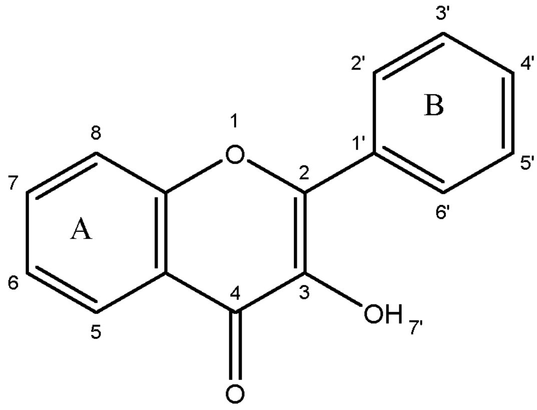

Flavonol

In the flavone derived condensed tannins, the base is usually hydroxylated in various positions and polymerized to a certain degree to obtain the high molecular weight polyphenol character.

Condensed tannins are found in virtually all the families of plants and comprise up to 50% of the dry weight of leaves.

The left ring in the flavonoid structure shown above (comprising carbon atoms 5 to 8) is usually referred to as the A-ring whereas the ring to the right (comprising carbon atoms 1' to 6') is usually referred to as the B-ring.

A first preferred group of condensed tannins suitable for the purpose of the present invention are flavan-3-ol-oligomers with 4→6 and/or 4→8 carbon linkages which may be represented by the following general fomula

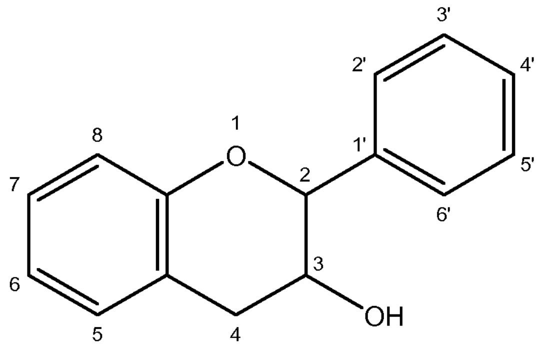

flavan-3-ol

and where the monomeric units are linked through carbon atoms 4 and 6 or 4 and 8, respectively.

In accordance with a preferred embodiment of the present invention the condensed tannins comprise monomeric units which comprise an A-ring derived from resorcinol or phloroglucinol and a B-ring derived from catechol or pyrogallol.

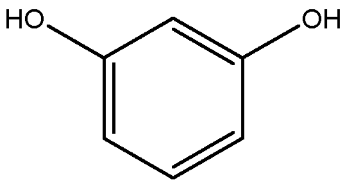

Resorcinol

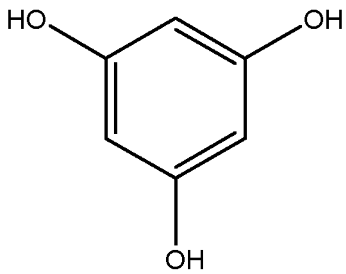

Phloroglucinol

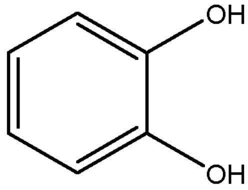

Catechol

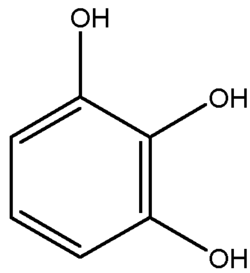

Pyrogallol

which yield basic structures for the monomeric units depicted below

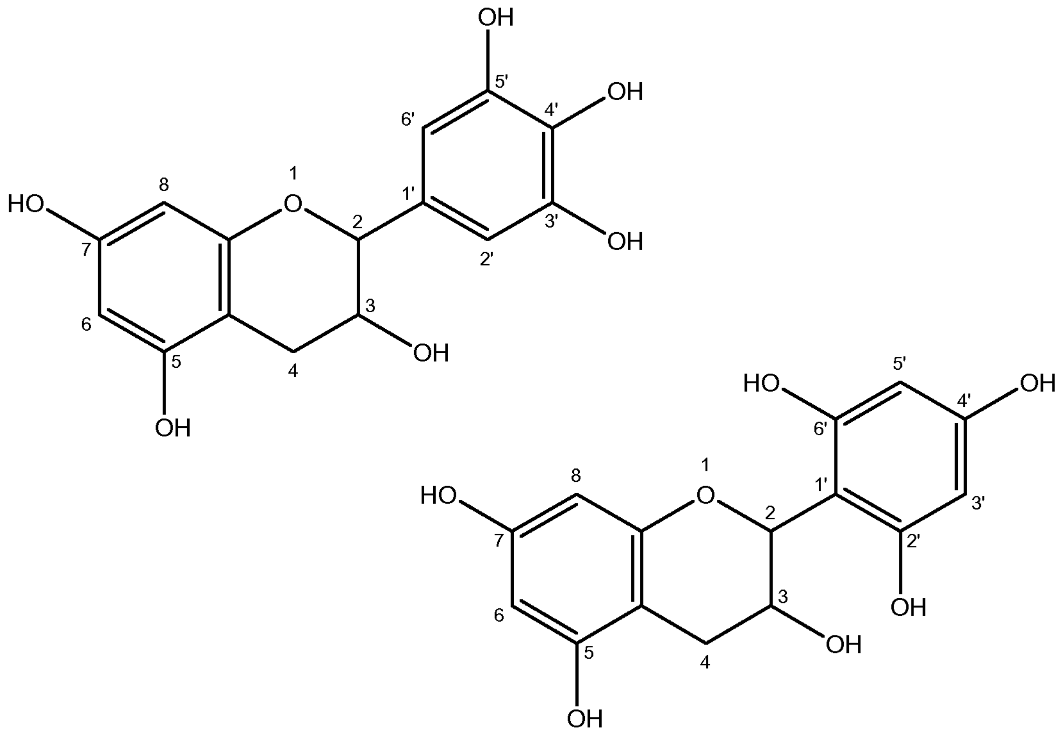

where the combination of resorcinol and phloroglucinol respectively pyrogallol is shown.

Some tannins respectively tannin extracts are commercially available. The most relevant are from Mimosa (*Acacia mearnsii* formerly *mollissima,* De Wildt), Quebracho (*Schinopsis balansae* and *Schinopsi lorentzii*) and Pine (*Pinus radiata* and *Pinus pinaster*) sources, with tannins or tannin extracts derived from pine and in particular from Pinus pinaster being particularly preferred in accordance with the present invention.

The term "average degree of substitution" as used herein depicts the average degree of replacement of phenolic hydroxyl groups by hydroxypropyl ether groups in the monomeric unit of the condensed tannin. It is readily apparent that the two preferred structures shown above have a maximum degree of substitution of 6 as the monomeric unit comprises six phenolic hydroxyl groups.

Accordingly, a "degree of substitution in the range of from 0.1 to 4.7" denotes monomeric units wherein on average 0.1 to 4.7 phenolic hydroxyl groups in the monomeric unit of the respective condensed tannin have been replaced by hydroxypropyl ether groups.

The data of the products obtainable by the reaction of bark extracts comprising condensed tannins with propylene oxide at the pH and temperature defined above appear to indicate that the products are quite homogenous as far as the degree of substitution is concerned. Homogeneity or "variation of the degree of substitution" is intended to denote the difference in the degree of substitution between the derivative with the highest degree of substitution and the derivative with the lowest degree of substitution in the sample. This parameter represents an indicator of the homogeneity of the degree of substitution in the reaction mixture after the derivatization. The data obtained indicate that the variation of the degree of substitution is often 0.6 and thus indicates that the maximum difference in the degree of substitution is 0.6

hydroxypropyl ether groups per monomeric unit in the sample.

The average degree of substitution as defined above in accordance with the present invention is determined by nuclear magnetic resonance spectroscopy (NMR) by relating the aliphatic signals at d:0.8-1.5 ppm to the B-ring aromatic signals at d: 6.5 to 7.5 ppm as reference according to equation 1.

$$DS = \frac{\int_{\delta=0.8\,ppm}^{\delta=1.5\,ppm} (-CH_3 \ or \ -OCOCH_3)}{\int_{\delta=6.5\,ppm}^{\delta=7.5\,ppm} (-CH_{aromatic(B-ring)})} \quad (1)$$

The variation of the degree of substitution is determined by separating the hydroxypropylated products into certain molecuklar weight fractions and determining the average degree of substitution for the isolated fractions. For the purpose of this invention a variation of the degree of substitution of 0.6 or less would mean that the average degree of substitution between any two separate fractions isolated does not exceed 0.6.

Preferred hydroxypropylated condensed tannin derivatives in accordance with the present invention have a variation of the degree of substitution of 0.5 or less, more preferably 0.3 or less when measured for at least three, preferably at least five different molecular weight fractions.

Dependent on the degree of substitution the hydroxypropylated condensed tannin derivatives in accordance with the present invention are useful for a large variety of uses. Whereas derivatives with a low degree of substitution, especially in the range of from 0.1 to 0.7 are useful as ingredients of systems for the synthesis of tannin derived foams, in particular of adhesive foams, derivatives with a comparatively high degree of substitution, preferably in the range of from 3.5 to 4.7 are particularly suitable for the manufacture of polyurethane resins. A third group of preferred hydroxypropylated condensed tannin derivatives in accordance with the present invention has a degree of substitution in the range of from 1.0 to 3.0. These derivatives could be useful for the preparation of aromatic polyesters.

For the first group with a degree of substitution in the range of from 0.1 to 0.8 there is a nearly linear relationship between the degree of substitution and the gelation time of solutions of these derivatives with formaldehyde. For a given pH of the solution the gelation time can be precisely tuned by adjusting the degree of substitution. The higher the degree of substitution, the higher the gelation time.

The glass transition temperature also shows a dependence on the degree of substitution and can thus be fine-tuned by adjusting same. With increasing degree of substitution there is a decrease of the glass transition temperature. Thus, by selecting the appropriate degree of substitution the skilled person will obtain derivatives with a glass transition temperature in a narrow range. Overall the glass transition temperature varies between 120 and 150°C for a degree of substitution in the range of from 4.7 to 0.1.

The normalized solubility of the hydroxypropylated condensed tannin derivatives in accordance with the present invention again is dependent on the degree of substitution and thus controlling this degree of substitution in a narrow range allows to adjust the solubility of the respective derivatives in accordance with the present invention. The solubility in water increases with decreasing degree of substitution whereas the solubility in ethanol not surprisingly shows an increase with increasing degree of substitution. The solubility in water decreases by a factor of approximately 5 with increasing degree of substitution, whereas the respective solubility in ethanol increases by a factor of nearly 20 with increasing degree of substitution.

The thermal stability of the hydroxypropylated condensed tannin derivatives in accordance with the present invention also increases with increasing degree of substitution, which effect can be determined by measuring the onset of decomposition by thermal gravimetric analysis.

The hydroxypropylated condensed tannin derivatives in accordance with the present invention can be preferably obtained by a process comprising the steps of

a) dissolving the bark extract comprising condensed tannins in an aqueous solvent at a pH in the range of from 9 to 14, preferably of form 10 to 14,

B) adding propylene oxide in an amount of from 0.1 to 6, preferably of from 0.3 to 5 moles per mole of tannin monomeric units,

c) reacting the mixture at a temperature in the range of from 10 to 50°C for at least 30 minutes, preferably at least 60 minutes and even more preferably for a period of from 6 to 48 hours,

) subsequently heating the reaction mixture obtained in step c) to a temperature of from 55 to 90°C, preferably of from 60 to 80°C, preferably for a period or from 5 minutes to 12 hours, preferably of from 20 minutes to 6 hours and

e) finally precipitating the product by lowering the pH to a value in the range of from 1 to 4, preferably in the

range of from 1.5 to 3.

The pH in the first step of the reaction can be adjusted by using a suitable base. By way of example, alkali metal hydroxides may be mentioned in this regard. The skilled person is aware of other suitable bases for adjusting the pH.

The reaction scheme in Fig. 1 shows the principal reaction of P. pinaster bark tannin with propylene oxide for the case of a C-15 flavonoid repeat unit being fully derivatized.

It has also been found in the course of the present invention that the polydispersity index (PDI) of the hydroxypropylated condensed tannin derivatives in accordance with the present inventions is significantly reduced in some cases compared to the respective index of the starting material which is an interesting feature for commercial uses of the products where normally, for homogeneity reasons a PDI as low as possible is desirable.

Whereas the PDI of the staring materials is often exceeding fifteen or even twenty, the hydroxypropylated products in accordance with the present invention often show PDI values significantly below 10 or even below 8.

It has surprisingly been found that by controlling the reaction conditions in the manner described above products can be obtained which have better reproducibility of properties as was obtained in the prior art.

Furthermore, it has also surprisingly been found that there is an almost linear relationship between the degree of substitution and the molar ratio of propylene oxide to monomeric unit of the condensed tannin. There is a nearly quantitative use of the propylene oxide in the course of the reaction. This suggests indiscriminate reaction of all available denoted hydroxy groups with propylene oxide in stoichiometric yields. This is a surprising result considering the mildness of the reaction conditions.

The hydroxypropylated condensed tannin derivatives in accordance with the present invention may be used as starting materials for the manufacture of foams or resins, in particular of substituted resins for phenol /formaldehyde resins or for polyurethanes.

Examples

**Materials.** Pine (*P. pinaster*) bark tannin was supplied by DTR (Dérivés Résiniques et Terpéniques) of Dax, France. This tannin is a mixture of oligomers with a significant abundance of trimers and tetramers that are mostly 4→8 linked. The samples were stored over silica gel in a vacuum desiccator and freeze-dried prior to use. All other reagents were used as received.

**Hydroxypropylation.** Tannin (100 g, ca. 80 mmol) was dissolved in 500 mL aq. 2N NaOH and the pH was adjusted to 12. One fourth, each, was combined with different molar equivalents of propylene oxide (PO). The PO/tannin monomeric unit (C-15) ratio ranged from 1.0 to 4.0. The reaction was carried out for 24 h while stirring at room temperature (~22 °C). Subsequently, the reaction mixture was heated to 60 °C for 30 min to remove potentially unreacted PO before it was allowed to cool. Adjusting the pH to 2 using conc. HCl (40% v/v) produced a precipitate that was centrifuged. The supernatant was collected by decantation, and the precipitate was washed five times with distilled water and freeze-dried.

The supernatant was recovered either by solvent evaporation (roto-evaporation at 40 °C) followed by repeated precipitation or by dialysis until the permeate proved free of chloride ions (nylon membranes by Serva Electrophoresis, USA). The purified supernatant was roto-evaporated at <60 °C and freeze-dried.

**Determination of degree of substitution (DS).** NMR spectra were used to quantify the DS of HPT and the respective acetates by relating the aliphatic signals at $\delta$: 0.8-1.5 ppm to the B-ring aromatic signals at $\delta$: 6.5-7.5 ppm as reference according to equation 1.

Three different fractions were obtained that represented an initial acid precipitate (fraction 1), an acid precipitate following concentration by solvent evaporation (fraction 2), and a dialysis retentate (fraction 3). The distinction of the three fractions was evaluated by $^1$H-NMR spectroscopy (Figure 3). When comparing the three fractions of HPT-preparations 1 and 4, it becomes apparent that the chemical structure of the fractions remains unchanged. Differences in isolation behaviour must be the result of molecular weight or concentration factors.

The aggregate yields of the initial precipitate fraction, fraction 1, with the fraction obtained by either evaporation (fraction 2) or dialysis (fraction 3), yield I and yield II, respectively, reveal that yields in excess of 80% by weight are obtained for all degree of substitution (DS)-levels (Figure 4). However, the data for the individual fractions vary significantly with PO/C-15 reaction ratio. Fraction 1-yields decline by 25% nearly linearly from 60 to 45%, as PO/C-15 ratio increases from 1.0 to 4.0. Yields of fractions 2 (from solvent evaporation) and 3 (from dialysis) increase conversely by about 1/3. This behavior is a reflection of changes in solubility characteristics of the propoxylated tannin derivatives in relation to an apparent DS resulting from the molar ratio of propylene oxide to monomeric unit of tannin. This change is remarkable in light of both ease and efficiency of chemical modification.

## Claims

**1.** Hydroxypropylated condensed tannin derivatives having an average degree of substitution of phenolic hydroxyl groups by hydroxypropyl ether groups in the monomeric unit in the range of from 0.1 to 4.7 obtainable by reacting a bark extract comprising condensed tannins in an aqueous solvent at a pH in the range of from 9 to 14 with propylene

oxide at a temperature in the range of from 10 to 50°C..

**2.** Hydroxypropylated condensed tannin derivatives in accordance with claim 1 or 2 having an average degree of substitution in the range of from 0.1 to 0.8.

**3.** Hydroxypropylated condensed tannin derivatives in accordance with claim 1 or 2 having an average degree of substitution in the range of from 1.0 to 3.5.

**4.** Hydroxypropylated condensed tannin derivatives in accordance with claim 1 or 2 having an average degree of substitution in the range of from 4.0 to 4.7.

**5.** Hydroxypropylated tannin derivatives in accordance with any of claims 1 to 5 derived from extracts obtained from pine bark.

**6.** Hydroxypropylated tannin derivatives in accordance with claim 6 wherein the pine is Pinus pinaster.

**7.** Use of hydroxypropylated condensed tannin derivatives in accordance with any of claims 1 to 7 for the manufacture of foams or resins.

**8.** Use of hydroxypropylated condensed tannin derivatives in accordance with claim 5 for the manufacture of polyurethane resins.

**9.** Use of hydroxypropylated condensed tannin derivatives in accordance with claim 3 for the manufacture of foams.

**10.** A process for the manufacture of hydroxypropylated condensed tannin derivatives comprising the steps

a) dissolving a berk extract comprising condensed tannins in an aqueous solvent at a pH in the range of from 9 to 14,
b) adding propylene oxide in an amount of from 0.1 to 6 moles per mole of tannin monomeric unit,
c) reacting the mixture at a temperature in the range of from 10 to 50 °C for at least 30 minutes,
d) optionally, subsequently heating the reaction mixture obtained in step c) to a temperature of from 55 to 90 °C and
e) finally precipitating the product by lowering the pH to a value in the range of from 1 to 4.

**11.** Process in accordance with claim 9 wherein the reaction time in step c) is in the range of from 6 to 48 h.

**12.** Process in accordance with claim 9 or 10 wherein the heating time in step d) is in the range of from 5 min to 12 h.

Fig. 1

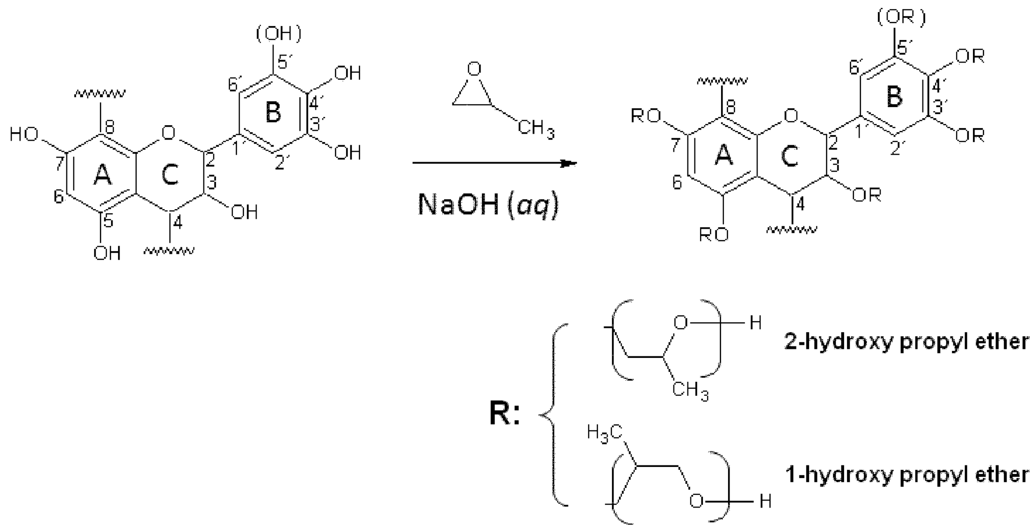

Fig. 2

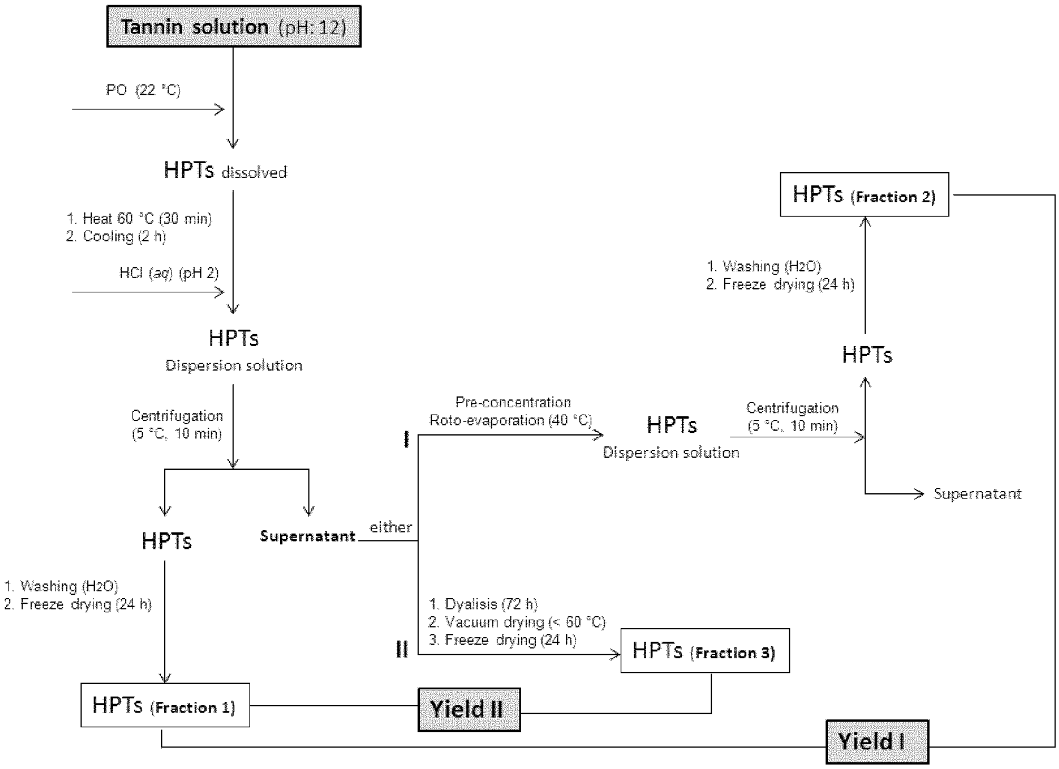
Tannin solution (pH: 12)
PO (22 °C)
HPTs dissolved
1. Heat 60 °C (30 min)
2. Cooling (2 h)
HCl (aq) (pH 2)
HPTs
Dispersion solution
Centrifugation
(5 °C, 10 min)
HPTs
Supernatant
either
I
Pre-concentration
Roto-evaporation (40 °C)
HPTs
Dispersion solution
Centrifugation
(5 °C, 10 min)
HPTs
Supernatant
1. Washing (H2O)
2. Freeze drying (24 h)
HPTs (Fraction 2)
II
1. Dyalisis (72 h)
2. Vacuum drying (< 60 °C)
3. Freeze drying (24 h)
HPTs (Fraction 3)
1. Washing (H2O)
2. Freeze drying (24 h)
HPTs (Fraction 1)
Yield II
Yield I

Fig. 3

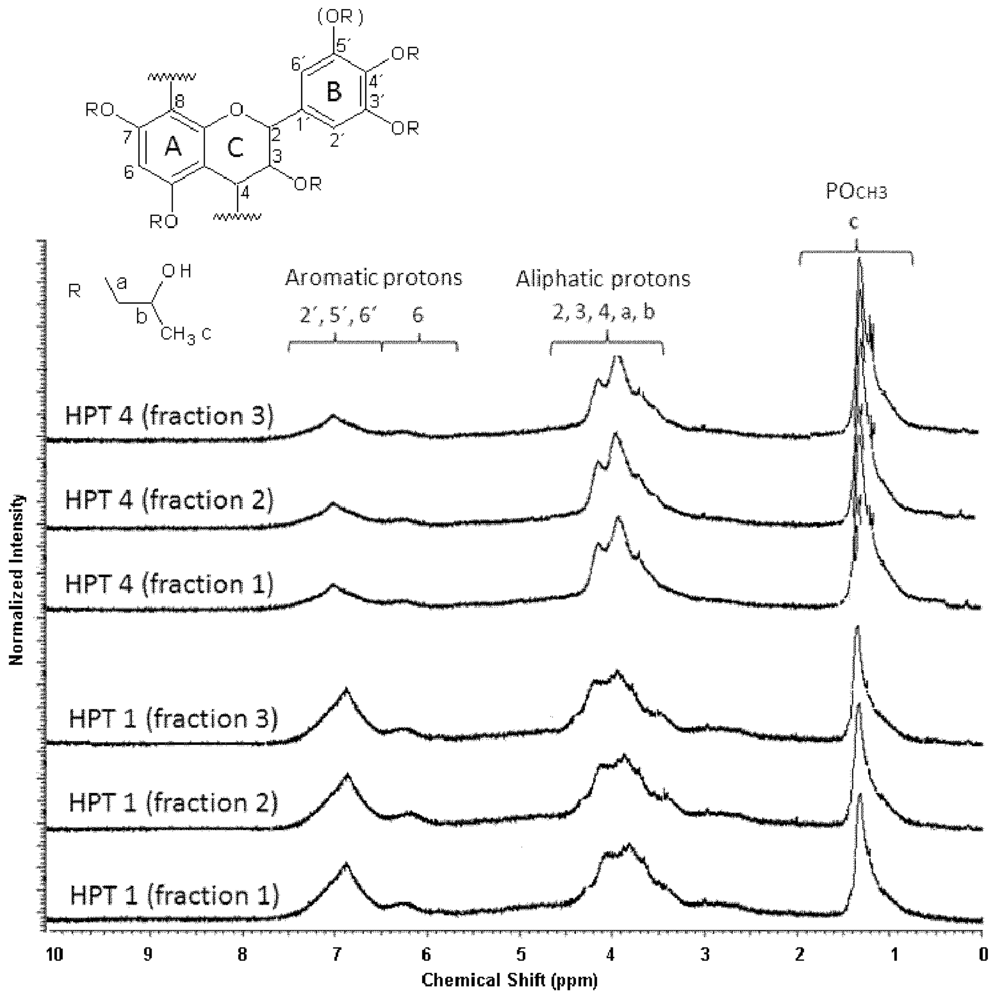
POCH3
c
Aromatic protons
2′, 5′, 6′
6
Aliphatic protons
2, 3, 4, a, b
HPT 4 (fraction 3)
HPT 4 (fraction 2)
HPT 4 (fraction 1)
HPT 1 (fraction 3)
HPT 1 (fraction 2)
HPT 1 (fraction 1)
Normalized Intensity
10
9
8
7
6
5
4
3
2
1
0
Chemical Shift (ppm)

Fig. 4

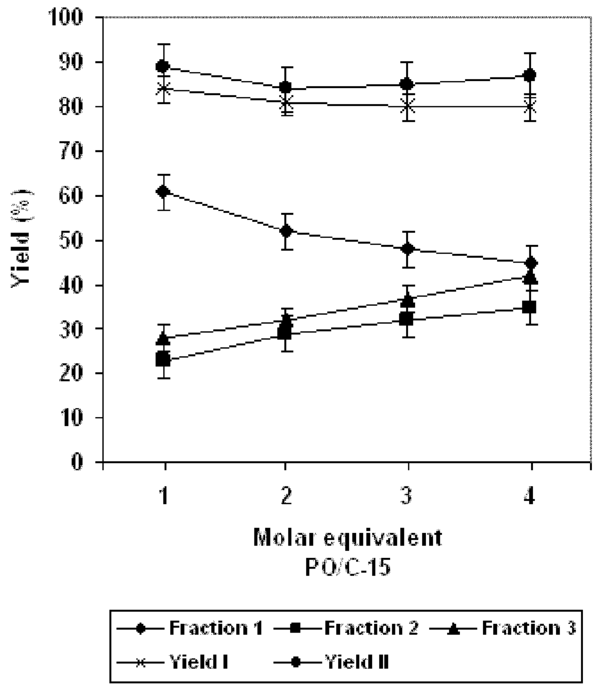
100
90
80
70
60
50
40
30
20
10
0
Yield (%)
1
2
3
4
Molar equivalent
PO/C-15
Fraction 1
Fraction 2
Fraction 3
Yield I
Yield II

Europäisches Patentamt
European Patent Office
Office européen des brevets

# EUROPEAN SEARCH REPORT

Application Number
EP 13 16 8572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | CAROLINA ANDREIA CATETO ET AL: "Optimization Study of Lignin Oxypropylation in View of the Preparation of Polyurethane Rigid Foams", INDUSTRIAL & ENGINEERING CHEMISTRY RESEARCH, vol. 48, no. 5, 4 March 2009 (2009-03-04), pages 2583-2589, XP055076453, ISSN: 0888-5885, DOI: 10.1021/ie801251r<br>* the whole document *<br>----- | 1-12 | INV.<br>C07D311/62<br>C08L75/04 |
| A,D | LUIS SERRANO ET AL: "Oxypropylation of Rapeseed Cake Residue Generated in the Biodiesel Production Process", INDUSTRIAL & ENGINEERING CHEMISTRY RESEARCH, vol. 49, no. 4, 17 February 2010 (2010-02-17), pages 1526-1529, XP055076450, ISSN: 0888-5885, DOI: 10.1021/ie9016732<br>* the whole document *<br>----- | 1-12 | |
| | | | TECHNICAL FIELDS SEARCHED (IPC) |
| A,D | GANDINI, ALESSANDRO ET AL: "Partial or total oxypropylation of natural polymers and the use of the ensuing materials as composites or polyol macromonomers", MONOMERS, POLYMERS AND COMPOSITES FROM RENEWABLE RESOURCES, 2008, pages 273-288, XP009172076, ELSEVIER LTD., OXFORD, UK. ISBN: 978-0-08-045316-3<br>* the whole document *<br>-----<br>-/-- | 1-12 | C07D<br>C08L |

The present search report has been drawn up for all claims

2

| Place of search | Date of completion of the search | Examiner |
|---|---|---|
| Munich | 28 August 2013 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 13 16 8572

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | NADJI, HAMID ET AL: "Oxypropylation of lignins and preparation of rigid polyurethane foams from the ensuing polyols", MACROMOLECULAR MATERIALS AND ENGINEERING , 290(10), 1009 1016 CODEN: MMENFA; ISSN: 1438-7492, 2005, XP55026228, DOI: 10.1016/B978-0-08-045316-3.00012-0 * the whole document * ----- | 1-12 | |
| X,P | DANNY E. GARCÍA ET AL: "Hydroxypropyl tannin derivatives from Pinus pinaster (Ait.) bark", INDUSTRIAL CROPS AND PRODUCTS, vol. 49, 1 August 2013 (2013-08-01), pages 730-739, XP055076375, ISSN: 0926-6690, DOI: 10.1016/j.indcrop.2013.06.019 * the whole document * ----- | 1-12 | |
| | | | TECHNICAL FIELDS SEARCHED (IPC) |

The present search report has been drawn up for all claims

2

| Place of search | Date of completion of the search | Examiner |
|---|---|---|
| Munich | 28 August 2013 | Klein, Didier |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................................................
& : member of the same patent family, corresponding document

EPO FORM 1503 03.82 (P04C01)